# EUROPÄISCHE PATENTSCHRIFT

(11) **EP 4 096 794 B1**
(45) Veröffentlichungstag und Bekanntmachung des Hinweises auf die Patenterteilung: **29.05.2024**
(21) Anmeldenummer: 21701944.7
(22) Anmeldetag: 18.01.2021
(51) Int. Cl.: A61Q 17/04, A61K 8/06, A61K 8/02, A61K 8/27, A61K 8/29, A61K 8/37, A61K 8/34

(54) **SONNENSCHUTZMITTEL MIT PIGMENT-FILTERSYSTEM**
SUNSCREEN WITH PIGMENT FILTER SYSTEM
ÉCRAN SOLAIRE AVEC SYSTÈME DE FILTRES PIGMENTAIRES

(30) Priorität: 29.01.2020 DE 102020201045
(43) Veröffentlichungstag der Anmeldung: 07.12.2022
(73) Patentinhaber: Beiersdorf AG, 22529 Hamburg (DE)
(72) Erfinder: KAACK, Jorina, 24558 Henstedt-Ulzburg (DE); SCHADE, Tatjana, 25866 Mildstedt/Rosendahl (DE); VON DER FECHT, Stephanie, 22880 Wedel (DE)
(74) Vertreter: Beiersdorf AG
(86) Internationale Anmeldenummer: PCT/EP2021/050894
(87) Internationale Veröffentlichungsnummer: WO 2021/151694

(56) Entgegenhaltungen:
- EP-A1- 1 762 217
- EP-A2- 2 465 486
- WO-A1-2019/050785
- WO-A2-2013/120829
- DE-A1-102005 005 176
- DE-A1-102007 005 093
- DE-A1-102007 005 335

## Beschreibung

Die vorliegende Erfindung betrifft eine kosmetische Wasser-in-ÖI-Emulsion (W/O-Emulsion) enthaltend
a) Titandioxid und/oder Zinkoxid mit einer Primärpartikelgröße zwischen 2 und 200 nm und
b) 1 bis 2,5 Gewichts-%, bezogen auf das Gesamtgewicht der Zubereitung, Polyglyceryl-3-Diisostearat (INCI: Polyglyceryl-3-Diisostearate).

Der Trend weg von der vornehmen Blässe hin zur "gesunden, sportlich braunen Haut" ist seit Jahren ungebrochen. Um diese zu erzielen setzen die Menschen ihre Haut der Sonnenstrahlung aus, da diese eine Pigmentbildung im Sinne einer Melaninbildung hervorruft. Die ultraviolette Strahlung des Sonnenlichtes hat jedoch auch eine schädigende Wirkung auf die Haut. Neben der akuten Schädigung (Sonnenbrand) treten Langzeitschäden wie ein erhöhtes Risiko an Hautkrebs zu erkranken bei übermäßiger Bestrahlung mit Licht aus dem UVB-Bereich (Wellenlänge: 280-320 nm) auf. Die übermäßige Einwirkung der UVB- und UVA-Strahlung (Wellenlänge: 320-400 nm) führt darüber hinaus zu einer Schwächung der elastischen und kollagenen Fasern des Bindegewebes. Dies führt zu zahlreichen phototoxischen und photoallergischen Reaktionen und hat eine vorzeitige Hautalterung zur Folge.

Zum Schutz der Haut wurden daher eine Reihe von Lichtschutzfiltersubstanzen entwickelt, die in kosmetischen Zubereitungen eingesetzt werden können. Diese UVA- und UVB-Filter sind in den meisten Industrieländern in Form von Positivlisten wie der Anlage 7 der deutschen Kosmetikverordnung zusammengefasst.

Eine besondere Form von UV-Lichtschutzfiltersubstanzen stellen die Mikropigmente dar. Die UV-Schutzwirkung der Mikropigmente beruht auf den physikalischen Effekten der Reflexion und Lichtstreuung. In kosmetischen Zubereitungen werden als Mikropigmente fast ausschließlich anorganische Mikropigmente aus Titandioxid, Zinkoxid oder Mischoxiden mit zum Beispiel Eisenoxiden eingesetzt.

Die Vorteile von Mikropigmenten als UV-Filtersubstanz in kosmetischen Zubereitungen liegen vor allem darin begründet, dass die Pigmente im Gegensatz zu gelöst oder flüssig vorliegenden organischen UV-Filtern, nicht in die Haut penetrieren können. Das Auftreten von allergischen Reaktionen ist damit ausgeschlossen.

WO2019/050785 A1 offenbart UV-Schutzzusammensetzung enthaltend hydrophob behandelte TiO2 und/oder ZnO Nano-Partikel. DE102007005335 A1 offenbart wässrige kosmetische Zubereitungen enthaltend partikuläre anorganische UV-Lichtschutzfilter mit einer durchschnittlichen Partikelgrösse von 10-500 nm. EP1762217 A1 offenbart Sonnenschutzmittel, welche auch anorganische Nanopigmente enthalten.

Nachteilig am Stande der Technik ist jedoch der Umstand, dass Mikropigmente nur schwer stabil in kosmetische Zubereitungen (insbesondere Emulsionen) einzuarbeiten sind. Insbesondere wenn Mikropigmente in höheren Konzentrationen (Konzentrationen über 7 Gewichts-%, bezogen auf das Gesamtgewicht der Zubereitung) eingesetzt werden, bilden sie relativ schnell Pigment-Agglomerate, die aus der Zubereitung ausfallen. Die Lager- und Temperaturstabilität der Zubereitung ist gering.

Nach dem Stande der Technik versucht man, diese Probleme mit Hilfe von Polyethylenglycol, Polyethylenglycolethern und Polyethylenglycolestern (sogenannten PEG-Derivaten) als Emulgatoren zu lösen. Derartige PEG-Derivate sind jedoch beim Verbraucher unbeliebt, weil ihre Verträglichkeit immer wieder diskutiert wird und sie die Penetration von Inhaltsstoffen in tiefere Hautschichten befördern können. Ob derartige Befürchtungen letztlich wissenschaftlich begründet sind oder nicht, kann im Rahmen der vorliegenden Offenbarung offen bleiben. Entscheidend ist der Wusch der Verbraucher nach PEG-freien Zubereitungen.

Außerdem versucht man nach dem Stande der Technik die Stabilitätsprobleme durch den Zusatz von Polymeren und Copolymeren der Acrylsäure (z.B. Carbomere, Acrylates/Alkylacrylates-Crosspolymer) oder Coplymeren des Vinylpyrrolidons in den Griff zu bekommen. Im Rahmen der sogenannten "Mikroplastik"-Diskussion wird allerdings der Einsatz auch dieser Stoffe zunehmend kritisch gesehen. Auch hier kann die Frage, ob der rückstandsfreie Abbau dieser Stoffe in der Natur und in Kläranlagen ein Problem darstellt, im Rahmen der vorliegenden Offenbarung offen bleiben. Entscheidend ist auch hier der Wunsch der Verbraucher nach Sonnenschutzprodukten, die er für "Mikroplastik-frei" hält.

Nachteilig an pigmenthaltigen W/O-Emulsionen des Standes der Technik sind nicht zuletzt deren sensorischen Eigenschaften. Meist hinterlassen derartige Zubereitungen auf der Haut einen fettig-öligen Eindruck.

Es war daher die Aufgabe der vorliegenden Erfindung, die Mängel des Standes der Technik zu beseitigen und gut verträgliche, pigmenthaltige W/O-Lichtschutzemulsionen von hoher Stabilität, guter Umweltverträglichkeit und angenehmen sensorischen Eigenschaften zu entwickeln. Die Formulierungen sollten ferner bei Ihrer Anwendung auf der Haut ein über einen längeren Anwendungszeitraum stabiles Absorptionsspektrum mit einer ausgewogenen UV-A/UV-B-Absorptionsbalance aufweisen und leicht verteilbar sein.

Überraschend gelöst werden die Aufgaben durch eine kosmetische Wasser-in-ÖI-Emulsion (W/O-Emulsion) gemäß Anspruch 1.

Es war dabei für den Fachmann nicht vorhersehbar, dass ausgerechnet diese geringe Menge an Emulgator Polyglyceryl-3-Diisostearat zu stabilen Emulsionen und darüber hinaus zu einer gleichmäßigen Pigmentverteilung auf der Haut führt, da der Fachmann zunächst davon ausgegangen wäre, dass eine höhere Stabilität nur durch eine höhere Emulgator-Konzentrationen zu erzielen ist. Höhere Emulgator-Konzentrationen wirken im vorliegenden Fall jedoch eher kontraproduktiv.

Dabei ist es erfindungsgemäß bevorzugt, wenn die kosmetische W/O-Emulsion keine organisch-chemischen UV-Filter enthält.

Es ist also insbesondere erfindungsgemäß bevorzugt, wenn die erfindungsgemäße W/O-Emulsion frei ist von UV-Filtern aus der Gruppe der Verbindungen Phenylen-1,4-bis-(2-benz-imidazyl)-3,3'-5,5'-tetrasulfonsäuresalze; 2-Phenylbenzimidazol-5-sulfonsäuresalze; 1,4-di(2-oxo-10-Sulfo-3-bornylidenmethyl)-Benzol und dessen Salze; 4-(2-Oxo-3-bornylidenmethyl)-benzolsulfonsäuresalze; 2-Methyl-5-(2-oxo-3-bornylidenmethyl)sulfonsäuresalze; 2,2'-Methylen-bis-(6-(2H-benzotriazol-2-yl)-4-(1,1,3,3-tetramethylbutyl)-phenol); 2-(2H-benzotriazol-2-yl)-4-methyl-6-[2-methyl-3-[1,3,3,3-tetramethyl-1-[(trimethylsilyl)oxy]disiloxanyl]propyl]-phenol; 3-(4-Methylbenzyliden)campher; 3-Benzylidencampher; Ethylhexylsalicylat; Terephthalidendicamphersulfonsäure; 4-(Dimethylamino)-benzoesäure(2-ethylhexyl)ester; 4-(Dimethylamino)benzoesäure-amylester; 4-Methoxybenzalmalon-säuredi(2-ethylhexyl)ester; 4-Methoxyzimtsäure(2-ethylhexyl)ester; 4-Methoxyzimtsäureisoamylester; 2-Hydroxy-4-methoxybenzophenon, 2-Hydroxy-4-methoxy-4'-methylbenzophenon; 2,2'-Dihydroxy-4-methoxybenzophenon; 2-(4'-Diethylamino-2'-hydoxybenzoyl)-benzoesäurehexylester, 4-(tert.-Butyl)-4'-methoxydibenzoylmethan; Homomenthylsalicylat; 2-Ethylhexyl-2-hydroxybenzoat; 2-Ethylhexyl-2-cyano-3,3-diphenylacrylat; Dimethicodiethylbenzalmalonat; 3-(4-(2,2-bis Ethoxycarbonylvinyl)-phenoxy)propenyl)-methoxysiloxan / Dimethylsiloxan - Copolymer; Dioctylbutylamidotriazon (INCI: Diethylhexyl-Butamidotriazone); 2,4-bis-[5-1(dimethylpropyl)benzoxazol-2-yl-(4-phenyl)-imino]-6-(2-ethylhexyl)-imino-1,3,5-triazin mit der (CAS Nr. 288254-16-0); 4,4',4"-(1,3,5-Triazin-2,4,6-triyltriimino)-tris-benzoesäure-tris(2-ethyl-hexylester) (auch: 2,4,6-Tris-[anilino-(p-carbo-2'-ethyl-1'-hexyloxy)]-1,3,5-triazin (INCI: Ethylhexyl Triazone); 2,4-Bis-{[4-(2-ethyl-hexyloxy)-2-hydroxy]-phenyl}-6-(4-methoxyphenyl)-1,3,5-triazin (INCI: Bis-Ethylhexyloxyphenol Methoxyphenyl Triazin); 2,4,6-Tris-(biphenyl)-1,3,5-triazin; 2,4-Bis-(4'- Di-neopentylaminobenzalmalonat)-6-(4"-butylaminobenzoat)-s-triazin, Merocyanine gewählt aus der Gruppe der Verbindungen

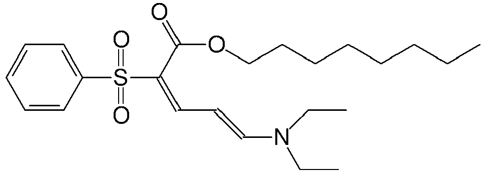

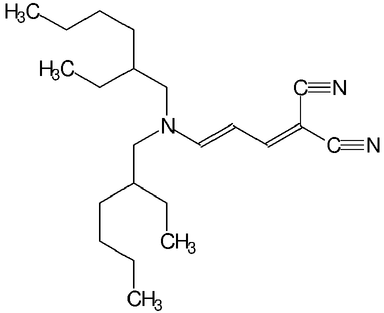

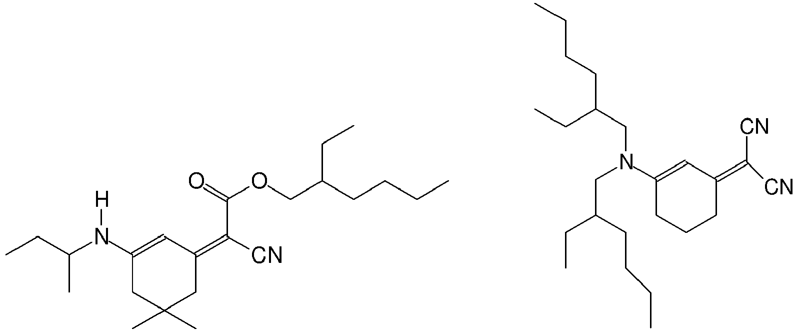

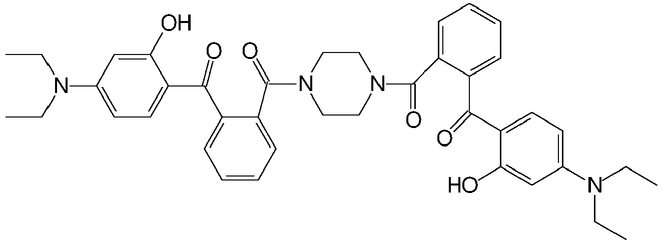

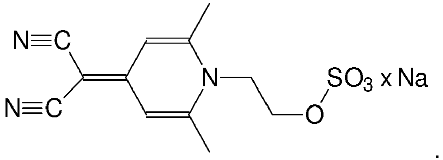

Erfindungsgemäß vorteilhafte Ausführungsformen der vorliegenden Erfindung sind ferner dadurch gekennzeichnet, dass die Zubereitung frei ist von Polymeren und Copolymeren der Acrylsäure und des Vinylpyrrolidons. Dies bedeutet, es ist erfindungsgemäß vorteilhaft, wenn die erfindungsgemäße W/O-Emulsion frei ist von Carbomeren, Acrylates/Alkylacrylates-Crosspolymer (z.B. Acrylates/C10-30Alkyl Acrylates Crosspolymer) oder Copolymeren des Vinylpyrrolidons wie VP/Hexadecene Copolymer.

Erfindungsgemäß vorteilhafte Ausführungsformen der vorliegenden Erfindung werden auch dadurch erhalten, dass die Zubereitung Polyglyceryl-4-diisostearat (INCI: Polyglyceryl-4 Diisostearate) enthält.

In einem solchen Falle ist es erfindungsgemäß bevorzugt, wenn die W/O-Emulsion Polyglyceryl-4 Diisostearate in einer Einsatzkonzentration von 0,5 bis 2,5 Gewichts-%, bezogen auf das Gesamtgewicht der Zubereitung, enthält.

Es ist erfindungsgemäß bevorzugt, wenn die erfindungsgemäße Zubereitung ausschließlich Titandioxid in der Kristallstruktur Rutil enthält. Dabei gilt erfindungsgemäß ein Titandioxid dann als ausschließlich in der Kristallstruktur vorliegend, wenn der Gehalt an anderen Kristallstrukturen in der Summe 1 Gewichts-%, bezogen auf die Gesamtmenge an Titandioxid, nicht überschreitet.

Es ist erfindungsgemäß bevorzugt, wenn die Primärpartikelgröße des erfindungsgemäßen Titandioxides im Bereich zwischen 5 und 50 nm liegt.

Erfindungsgemäß vorteilhafte Ausführungsformen der vorliegenden Erfindung sind dabei dadurch gekennzeichnet, dass das Titandioxid mit Silica (Siliziumdioxid und/oder Kieselsäure) beschichtet ist.

Es ist dabei erfindungsgemäß bevorzugt, wenn das mit Silica beschichtete Titandioxid auf der äußeren Seite der Silica-Schicht (d.h. auf der dem Titandioxid-abgewandten Seite) eine Schicht aus Dimethicone, Simethicone oder Methicone aufweist. Unter diesen Stoffen ist Dimethicone dabei erfindungsgemäß besonders bevorzugt.

Es ist erfindungsgemäß vorteilhaft, wenn die sekundäre Partikelgröße des Titandioxid in der Kristallstruktur Rutil mit einer Primärpartikelgröße von 2 -100 nm zwischen 0,05 und 50 µm beträgt. Dabei ist der Bereich von 0,1 bis 1 µm erfindungsgemäß bevorzugt.

Es ist erfindungsgemäß vorteilhaft, wenn die sekundäre Partikelgröße des Titandioxid in der Kristallstruktur Rutil mit einer Primärpartikelgröße von 2 -100 nm zwischen 0,05 und 50 µm beträgt. Dabei ist der Bereich von 0,1 bis 1 µm erfindungsgemäß bevorzugt.

Die primäre und sekundäre Partikelgröße entnimmt der Fachmann folgender Literaturstelle: SCCS/1516/13 Opinion on Titanium Dioxide (nano form) Colipa No S75 der Cosmetics Europe personal care association. Entsprechende Veröffentlichungen gibt es auch zur Primärpartikelgröße von Zinkoxid (SCCS/1489/12 Opinion).

Als Zinkoxid werden bevorzugt Partikel eingesetzt, deren Primärpartikelgröße kleiner 150 nm beträgt. Es ist erfindungsgemäß vorteilhaft mit Triethoxycaprylylsilane beschichtet.

Erfindungsgemäß vorteilhafte Ausführungsformen der vorliegenden Erfindung werden auch dadurch erhalten, dass die Zubereitung frei ist von Milchsäure und deren Salzen (Lactaten).

Hingegen ist es vorteilhaft im Sinne der vorliegenden Erfindung, wenn die erfindungsgemäße Emulsion Ethanol enthält. In einem solchen Falle beträgt die erfindungsgemäß vorteilhafte Einsatzkonzentration von 0,1 bis 5 Gewichts-%, bezogen auf das Gesamtgewicht der Zubereitung.

Darüber hinaus ist es erfindungsgemäß von Vorteil, wenn die erfindungsgemäße W/O-Emulsion eine oder mehrere Öle gewählt aus der Gruppe der Verbindungen Isopropylpalmitat, C12-15 Alkylbenzoat, Dicaprylylether enthält.

Der erfindungsgemäß vorteilhafte Konzentrationsbereich für Isopropylpalmitat beträgt dabei von 0,1 bis 30 Gewichts-%, bezogen auf das Gesamtgewicht der Zubereitung.

Der erfindungsgemäß vorteilhafte Konzentrationsbereich für C12-15 Alkylbenzoat beträgt dabei von 0,1 bis 10 Gewichts-%, bezogen auf das Gesamtgewicht der Zubereitung.

Der erfindungsgemäß vorteilhafte Konzentrationsbereich für Dicaprylylether beträgt dabei von 0,1 bis 30 Gewichts-%, bezogen auf das Gesamtgewicht der Zubereitung.

Erfindungsgemäß vorteilhafte Ausführungsformen der vorliegenden Erfindung sind dadurch gekennzeichnet, dass die Zubereitung hydriertes Rapsöl (INCI: Hydrogenated Rapeseed Oil) enthält. Der erfindungsgemäß vorteilhafte Konzentrationsbereich für hydriertes Rapsöl beträgt dabei von 0,1 bis 2 Gewichts-%, bezogen auf das Gesamtgewicht der Zubereitung.

Darüber hinaus kann die erfindungsgemäße W/O-Emulsion die für kosmetische Emulsionen üblichen zusätzlichen Inhaltsstoffe enthalten, beispielsweise Glycerin und Wasser, Parfümstoffe und Wirkstoffe wie Tocopherylacetat, Tocopherol und/oder Glycyrrhetinsäure.

Auch hat sich ein Gehalt an Magnesiumsulfat (INCI: Magnessium Sulfate) als erfindungsgemäß vorteilhaft herausgestellt. Für diesen Stoff beträgt die erfindungsgemäß vorteilhafte Einsatzkonzentration von 0,1 bis 8 Gewichts-%, bezogen auf das Gesamtgewicht der Zubereitung.

### Vergleichsversuch

Es wurden die folgenden drei Emulsion mit unterschiedlichem Gehalt an Polyglyceryl-3 Diisostearate hergestellt und ihre Lagerstabilität bei 40 °C Lagertemperatur untersucht.

| | **#1** | **#2** | **#3** |
|---|---|---|---|
| **INCI** | **m [%]** | **m [%]** | **m [%]** |
| Persea Gratissima Oil | 0,10 | 0,10 | 0,10 |
| Isopropyl Palmitate | 13,00 | 13,00 | 13,00 |
| C12-15 Alkyl Benzoate | 7,00 | 6,00 | 8,00 |
| Dicaprylyl Ether | 6,90 | 6,90 | 6,90 |
| Hydrogenated Rapeseed Oil | 1,00 | 1,00 | 1,00 |
| **Polyglyceryl-3 Diisostearate** | **2,00** | **3,00** | **1,00** |
| Polyglyceryl-4 Diisostearate/Polyhydroxystearate/Sebacate | 3,50 | 3,50 | 3,50 |
| Glycerin | 1,00 | 1,00 | 1,00 |
| Phenoxyethanol | 0,80 | 0,80 | 0,80 |
| Aqua | 31,00 | 31,00 | 31,00 |
| Alcohol Denat. + Aqua | 3,00 | 3,00 | 3,00 |
| Magnesium Sulfate | 0,70 | 0,70 | 0,70 |
| Zinc Oxide (nano) + Triethoxycaprylylsilane | 23,00 | 23,00 | 23,00 |
| Titanium Dioxide (nano) + Silica + Dimethicone | 7,00 | 7,00 | 7,00 |
| Summen: | 100,00 | 100,00 | 100,00 |

Ergebnis: Die Emulsionen zeigten nach 120 Tagen Lagerung bei 40°C ein unterschiedliches Bild. Während die Rezepturen #1 und #3 stabil waren, war Rezeptur #2 instabil und wies eine Ölabscheidung auf.

Fazit: Eine lagerstabile, homogene Emulsion mit hohem Pigmentgehalt kann nur erzielt werden, wenn der Gehalt an Polygylceryl-3 Diisostearat in der Zubereitung 2,5 Gewichts-% nicht überschreitet.

### Beispiele

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

| | **Bsp.1** | **Bsp. 2** | **Bsp. 3** | **Bsp. 4** | **Bsp.5** |
|---|---|---|---|---|---|
| **INCI** | **m [%]** | **m [%]** | **m [%]** | **m [%]** | **m [%]** |
| Persea Gratissima Oil | 0,10 | ⁰,10 | 0,10 | 0,10 | 0,10 |
| Isopropyl Palmitate | 13,00 | 13,00 | 13,00 | 13,00 | 15,00 |
| C12-15 Alkyl Benzoate | 7,00 | 8,00 | 6,50 | 8,00 | 5,00 |
| Dicaprylyl Ether | 6,90 | 6,90 | 6,90 | 12,40 | 18,90 |
| Hydrogenated Rapeseed Oil | 1,00 | 1,00 | 1,00 | 1,00 | 1,00 |
| **Polyglyceryl-3 Diisostearate** | 2,00 | 1,00 | 2,50 | 2,50 | 1,00 |
| Polyglyceryl-4 Diisostearate/Polyhydroxystearate/Sebacate | 3,50 | 3,50 | 3,50 | 3,50 | 3,50 |
| Glycerin | 1,00 | 1,00 | 1,00 | 1,00 | 1,00 |
| Phenoxyethanol | 0,80 | 0,80 | 0,80 | 0,80 | 0,80 |
| Aqua | 31,00 | 31,00 | 31,00 | 31,00 | 31,00 |
| Alcohol Denat. + Aqua | 3,00 | 3,00 | 3,00 | 3,00 | 3,00 |
| Magnesium Sulfate | 0,70 | 0,70 | 0,70 | 0,70 | 0,70 |
| Zinc Oxide (nano) + Triethoxycaprylylsilane | 23,00 | 23,00 | 23,00 | 20,00 | 15,00 |
| Titanium Dioxide (nano) + Silica + Dimethicone | 7,00 | 7,00 | 7,00 | 3,00 | 4,00 |
| **Summen:** | 100,00 | 100,00 | 100,00 | 100,00 | 100,00 |

## Patentansprüche

1. Kosmetische Wasser-in-Öl-Emulsion (W/O-Emulsion) enthaltend
a) Titandioxid und/oder Zinkoxid mit einer Primärpartikelgröße zwischen 2 und 200 nm,
b) 1 bis 2,5 Gewichts-%, bezogen auf das Gesamtgewicht der Zubereitung, Polyglyceryl-3-Diisostearat (INCI: Polyglyceryl-3-Diisostearate), wobei die Zubereitung über 7 Gewichts-% an Mikropigmenten, bezogen auf das Gesamtgewicht der Zubereitung, enthält, **dadurch gekennzeichnet, dass** die Zubereitung frei ist von Parabenen, Methylisothiazolinon, Chlormethylisothiazolinon und DMDM-Hydantoin, sowie frei ist von Polyethylenglycol, Polyethylenglycolethern und Polyethylenglycolestern (sogenannten PEG-Derivaten).

2. Kosmetische W/O-Emulsion nach Anspruch 1, **dadurch gekennzeichnet, dass** die Zubereitung keine organisch-chemischen UV-Filter enthält.

3. Kosmetische W/O-Emulsion nach einem der vorhergehenden Ansprüche, **dadurch gekennzeichnet, dass** die Zubereitung frei ist von UV-Filtern aus der Gruppe der Verbindungen Phenylen-1,4-bis-(2-benzimidazyl)-3,3'-5,5'-tetrasulfonsäuresalze; 2-Phenylbenzimidazol-5-sulfonsäuresalze; 1,4-di(2-oxo-10-Sulfo-3-bornylidenmethyl)-Benzol und dessen Salze; 4-(2-Oxo-3-bornylidenmethyl)benzolsulfonsäuresalze; 2-Methyl-5-(2-oxo-3-bornylidenmethyl)sulfonsäuresalze; 2,2'-Methylen-bis-(6-(2H-benzotriazol-2-yl)-4-(1,1,3,3-tetramethylbutyl)-phenol); 2-(2H-benzotriazol-2-yl)-4-methyl-6-[2-methyl-3-[1,3,3,3-tetramethyl-1-[(trimethylsilyl)oxy]disiloxanyl]propyl]-phenol; 3-(4-Methylbenzyliden)campher; 3-Benzylidencampher; Ethylhexylsalicylat; Terephthalidendicamphersulfonsäure; 4-(Dimethylamino)-benzoesäure(2-ethylhexyl)-ester; 4-(Dimethylamino)benzoesäure-amylester; 4-Methoxybenzalmalon-säuredi(2-ethylhexyl)ester; 4-Methoxyzimtsäure(2-ethylhexyl)ester; 4-Methoxyzimtsäureisoamylester; 2-Hydroxy-4-methoxybenzophenon, 2-Hydroxy-4-methoxy-4'-methylbenzophenon; 2,2'-Dihydroxy-4-methoxybenzophenon; 2-(4'-Diethylamino-2'-hydoxybenzoyl)-benzoesäurehexylester, 4-(tert.-Butyl)-4'-methoxydibenzoylmethan; Homomenthylsalicylat; 2-Ethylhexyl-2-hydroxybenzoat; 2-Ethylhexyl-2-cyano-3,3-di-phenylacrylat; Dimethicodiethylbenzalmalonat; 3-(4-(2,2-bis Ethoxycarbonylvinyl)-phenoxy)propenyl)-methoxysiloxan / Dimethylsiloxan - Copolymer; Dioctylbutylamidotriazon (INCI: Diethylhexyl-Butamidotriazone); 2,4-bis-[5-1(dimethyl-propyl)benzoxazol-2-yl-(4-phenyl)-imino]-6-(2-ethylhexyl)-imino-1,3,5-triazin mit der (CAS Nr. 288254-16-0); 4,4',4"-(1,3,5-Triazin-2,4,6-triyltriimino)-tris-benzoesäure-tris-(2-ethylhexylester) (auch: 2,4,6-Tris-[anilino-(p-carbo-2'-ethyl-1'-hexyloxy)]-1,3,5-triazin (INCI: Ethylhexyl Triazone); 2,4-Bis-{[4-(2-ethyl-hexyloxy)-2-hydroxy]-phenyl}-6-(4-methoxyphenyl)-1,3,5-triazin (INCI: Bis-Ethylhexyloxyphenol Methoxyphenyl Triazin); 2,4,6-Tris-(biphenyl)-1,3,5-triazin; 2,4-Bis-(4'- Di-neopentylaminobenzalmalonat)-6-(4"-butylaminobenzoat)-s-triazin, Merocyanine gewählt aus der Gruppe der Verbindungen

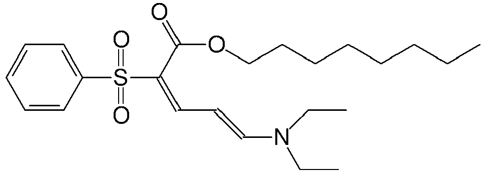

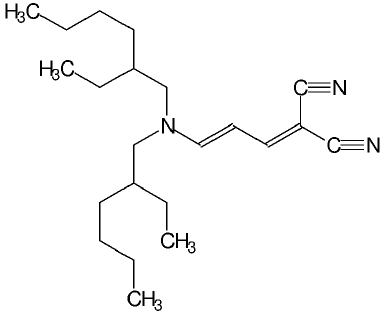

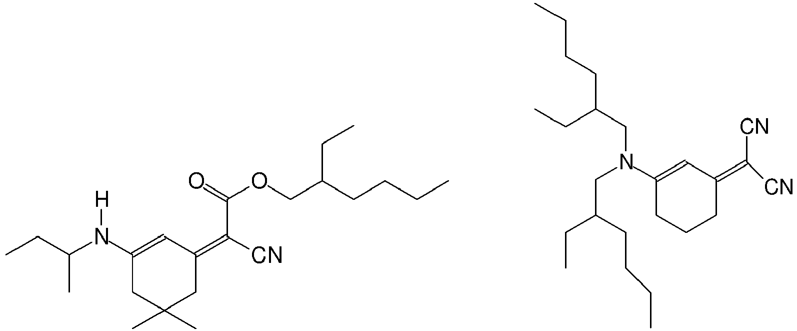

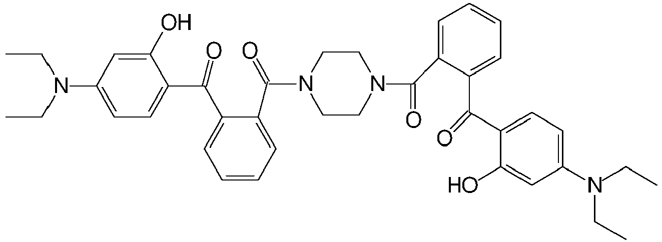

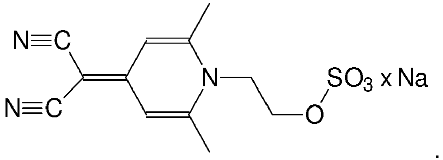

4. Kosmetische Emulsion nach einem der vorhergehenden Ansprüche, **dadurch gekennzeichnet, dass** die Zubereitung frei ist von Polymeren und Copolymeren der Acrylsäure und des Vinylpyrrolidons.

5. Kosmetische Emulsion nach einem der vorhergehenden Ansprüche, **dadurch gekennzeichnet, dass** die Zubereitung Polyglyceryl-4 Diisostearate enthält.

6. Kosmetische Zubereitung nach einem der vorhergehenden Ansprüche, **dadurch gekennzeichnet, dass** die Zubereitung frei ist von Milchsäure und deren Salzen (Lactaten).

7. Kosmetische Emulsion nach einem der vorhergehenden Ansprüche, **dadurch gekennzeichnet, dass** die Zubereitung Ethanol enthält.

8. Kosmetische Emulsion nach einem der vorhergehenden Ansprüche, **dadurch gekennzeichnet, dass** die Zubereitung eine oder mehrere Öle gewählt aus der Gruppe der Verbindungen Isopropylpalmitat, C12-15 Alkylbenzoat, Dicaprylylether enthält.

9. Kosmetische Emulsion nach einem der vorhergehenden Ansprüche, **dadurch gekennzeichnet, dass** die Zubereitung hydriertes Rapsöl (INCI: Hydrogenated Rapeseed Oil) enthält.

10. Kosmetische Emulsion nach einem der vorhergehenden Ansprüche, **dadurch gekennzeichnet, dass** die Zubereitung Titandioxid enthält, welches mit Silica (Siliziumdioxid und/oder Kieselsäure) beschichtet ist, welches auf der äußeren Seite der Silica-Schicht (d.h. auf der dem Titandioxid-abgewandten Seite) eine Schicht aus Dimethicone, Simethicone oder Methicone aufweist.

11. Kosmetische Emulsion nach einem der vorhergehenden Ansprüche, **dadurch gekennzeichnet, dass** das eingesetzte Titandioxid in der Kristallstruktur Rutil vorliegt.

12. Kosmetische Emulsion nach einem der vorhergehenden Ansprüche, **dadurch gekennzeichnet, dass** das Zinkoxid mit Triethoxycaprylylsilane beschichtet ist.

13. Kosmetische Emulsion nach einem der vorhergehenden Ansprüche, **dadurch gekennzeichnet, dass** die Zubereitung eine Mischung aus Titandioxid und Zinkoxid enthält.

14. Kosmetische Emulsion nach einem der vorhergehenden Ansprüche, **dadurch gekennzeichnet, dass** das Titandioxid eine Primärpartikelgröße von 2 -100 nm und aufweist.

15. Kosmetische Emulsion nach einem der vorhergehenden Ansprüche, **dadurch gekennzeichnet, dass** das Zinkoxid eine Primärpartikelgröße von weniger als 150 nm aufweist.

## Claims

1. Cosmetic water-in-oil emulsion (W/O emulsion) comprising
a) titanium dioxide and/or zinc oxide with a primary particle size between 2 and 200 nm,
b) 1% to 2.5% by weight, based on the total weight of the preparation, of Polyglyceryl-3 Diisostearate (INCI: Polyglyceryl-3 Diisostearate), the preparation comprising over 7% by weight of micropigments, based on the total weight of the preparation, **characterized in that** the preparation is free of parabens, methylisothiazolinone, chloromethylisothiazolinone and DMDM hydantoin, and is free of polyethylene glycol, polyethylene glycol ethers and polyethylene glycol esters (so-called PEG derivatives).

2. Cosmetic W/O emulsion according to Claim 1, **characterized in that** the preparation does not comprise any organochemical UV filters.

3. Cosmetic W/O emulsion according to either of the preceding claims, **characterized in that** the preparation is free of UV filters from the following group of compounds: phenylene-1,4-bis(2-benzimidazyl)-3,3'-5,5'-tetrasulfonic acid salts; 2-phenylbenzimidazole-5-sulfonic acid salts; 1,4-di(2-oxo-10-sulfo-3-bornylidenemethyl)benzene and salts thereof; 4-(2-oxo-3-bornylidenemethyl)benzenesulfonic acid salts; 2-methyl-5-(2-oxo-3-bornylidenemethyl)sulfonic acid salts; 2,2'-methylenebis(6-(2H-benzotriazol-2-yl)-4-(1,1,3,3-tetramethylbutyl)phenol); 2-(2H-benzotriazol-2-yl)-4-methyl-6-[2-methyl-3-[1,3,3,3-tetramethyl-1-[(trimethylsilyl)oxy]disiloxanyl]propyl]phenol; 3-(4-methylbenzylidene)camphor; 3-benzylidenecamphor; ethylhexyl salicylate; terephthalidenedicamphorsulfonic acid; 2-ethylhexyl 4-(dimethylamino)benzoate; amyl 4-(dimethylamino)benzoate; di(2-ethylhexyl) 4-methoxybenzalmalonate; 2-ethylhexyl 4-methoxycinnamate; isoamyl 4-methoxycinnamate; 2-hydroxy-4-methoxybenzophenone; 2-hydroxy-4-methoxy-4'-methylbenzophenone; 2,2'-dihydroxy-4-methoxybenzophenone; hexyl 2-(4'-diethylamino-2'-hydroxybenzoyl)benzoate; 4-(tert-butyl)-4'-methoxydibenzoylmethane; homomenthyl salicylate; 2-ethylhexyl 2-hydroxybenzoate; 2-ethylhexyl 2-cyano-3,3-diphenylacrylate; dimethicodiethylbenzalmalonate; 3-(4-(2,2-bisethoxycarbonylvinyl)phenoxy)propenyl)methoxysiloxane / dimethylsiloxane copolymer; dioctylbutylamidotriazone (INCI: Diethylhexyl Butamido Triazone); 2,4-bis[5-1(dimethylpropyl)benzoxazol-2-yl-(4-phenyl)imino]-6-(2-ethylhexyl)imino-1,3,5-triazine with (CAS No. 288254-16-0); tris(2-ethylhexyl) 4,4',4"-(1,3,5-triazine-2,4,6-triyltriimino)trisbenzoate (also: 2,4,6-tris[anilino-(p-carbo-2'-ethyl-1'-hexyloxy)]-1,3,5-triazine (INCI: Ethylhexyl Triazone); 2,4-bis{[4-(2-ethylhexyloxy)-2-hydroxy]phenyl}-6-(4-methoxyphenyl)-1,3,5-triazine (INCI: Bis-Ethylhexyloxyphenol Methoxyphenyl Triazine); 2,4,6-tris(biphenyl)-1,3,5-triazine; 2,4-bis(4'-dineopentylaminobenzalmalonate)-6-(4"-butylaminobenzoate)-s-triazine; merocyanines selected from the following group of compounds:

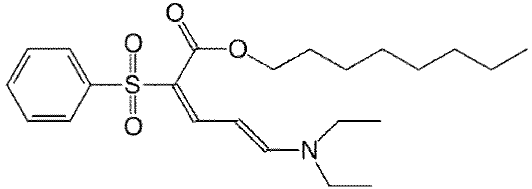

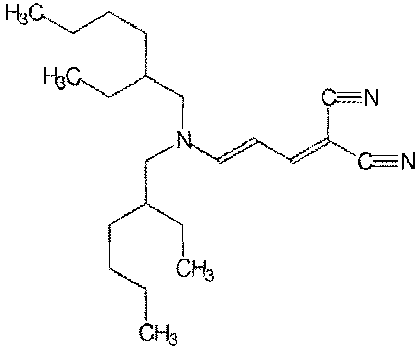

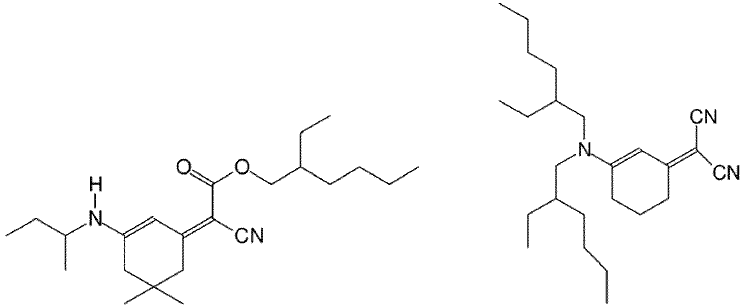

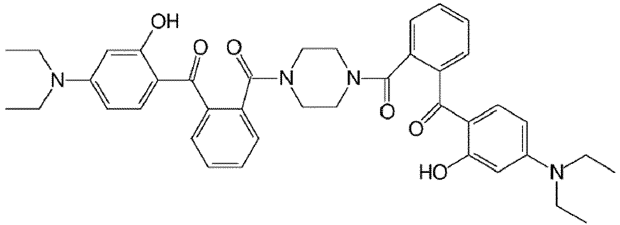

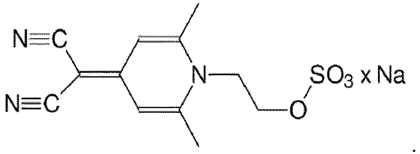

4. Cosmetic emulsion according to any of the preceding claims,
**characterized in that** the preparation is free of polymers and copolymers of acrylic acid and of vinylpyrrolidone.

5. Cosmetic emulsion according to any of the preceding claims,
**characterized in that** the preparation comprises Polyglyceryl-4 Diisostearate.

6. Cosmetic preparation according to any of the preceding claims,
**characterized in that** the preparation is free of lactic acid and salts thereof (lactates).

7. Cosmetic emulsion according to any of the preceding claims, **characterized in that** the preparation comprises ethanol.

8. Cosmetic emulsion according to any of the preceding claims,
**characterized in that** the preparation comprises one or more oils selected from the following group of compounds: isopropyl palmitate, C12-15 alkyl benzoate, dicaprylyl ether.

9. Cosmetic emulsion according to any of the preceding claims,
**characterized in that** the preparation comprises hydrogenated rapeseed oil (INCI: Hydrogenated Rapeseed Oil).

10. Cosmetic emulsion according to any of the preceding claims,
**characterized in that** the preparation comprises titanium dioxide, which is coated with silica (silicon dioxide and/or silicic acid), which has a layer of dimethicone, simethicone or methicone on the outer side of the silica layer (i.e. on the side facing away from the titanium dioxide).

11. Cosmetic emulsion according to any of the preceding claims,
**characterized in that** the titanium dioxide used is in the rutile crystal structure.

12. Cosmetic emulsion according to any of the preceding claims,
**characterized in that** the zinc oxide is coated with Triethoxycaprylylsilane.

13. Cosmetic emulsion according to any of the preceding claims,
**characterized in that** the preparation comprises a mixture of titanium dioxide and zinc oxide.

14. Cosmetic emulsion according to any of the preceding claims,
**characterized in that** the titanium dioxide has a primary particle size of 2-100 nm and.

15. Cosmetic emulsion according to any of the preceding claims,
**characterized in that** the zinc oxide has a primary particle size of less than 150 nm.

## Revendications

1. Émulsion cosmétique de type huileux (émulsion W/O) contenant
a) du dioxyde de titane et/ou de l'oxyde de zinc ayant une granulométrie primaire entre 2 et 200 nm,
b) 1 à 2,5 % en poids, par rapport au poids total de la préparation, de diisostéarate de polyglycéryle-3 (INCI : Polyglyceryl-3-Diisostearate), la composition contenant plus de 7 % en poids de micropigments, par rapport au poids total de la préparation, **caractérisée en ce que** la préparation est exempte de parabens, de méthylisothiazolinone, de chlorométhylisothiazolinone et de DMDM-hydantoïne, et est également exempte de polyéthylèneglycol, d'éthers de polyéthylèneglycol et d'esters de polyéthylèneglycol (appelés dérivés de PEG).

2. Émulsion cosmétique W/O selon la revendication 1, **caractérisée en ce que** la préparation ne contient pas de filtres UV organochimiques.

3. Préparation cosmétique W/O selon l'une des revendications précédentes, **caractérisée en ce que** la préparation est exempte de filtres UV du groupe des composés sels de l'acide phénylène-1 ,4-bis-(2-benzimidazyl)-3,3'-5,5'-tétrasulfonique ; sels de l'acide 2-phénylbenzimidazole-5-sulfonique ; 1,4-di(2-oxo-10-sulfo-3-bornylidèneméthyl)-benzène et ses sels ; sels de l'acide 4-(2-oxo-3-bornylidèneméthyl)benzènesulfonique ; sels de l'acide 2-méthyl-5-(2-oxo-3-bornylidèneméthyl)sulfonique ; 2,2'-méthylène-bis-(6-(2H-benzotriazol-2-yl)-4-(1,1,3,3-tétraméthylbutyl)-phénol) ; 2-(2H-benzotriazol-2-yl)-4-méthyl-6-[2-méthyl-3-[1,3,3,3-tétraméthyl-1-[(triméthylsilyl)oxy]disiloxanyl]propyl]-phénol ; 3-(4-méthylbenzylidène)camphre ; 3-benzylidènecamphre ; salicylate d'éthylhexyle ; acide téréphtalidènedicamphosulfonique ; ester 2-éthylhexylique de l'acide 4-(diméthylamino)-benzoïque ; ester amylique de l'acide 4-(diméthylamino)benzoïque ; ester 2-éthylhexylique de l'acide 4-méthoxybenzalmalonique ; ester 2-éthylhexylique de l'acide 4-méthoxycinnamique ; ester isoamylique de l'acide 4-méthoxycinnamique ; 2-hydroxy-4-méthoxybenzophénone, 2-hydroxy-4-méthoxy-4'-méthylbenzophénone ; 2,2'-dihydroxy-4-méthoxybenzophénone ; ester hexylique de l'acide 2-(4'-diéthylamino-2'-hydroxybenzoyl)-benzoïque ; 4-(tert.-butyl)-4'-méthoxydibenzoylméthane ; salicylate d'homomenthyle ; 2-hydroxybenzoate de 2-éthylhexyle ; acrylate de 2-éthylhexyl-2-cyano-3,3-di-phényle ; malonate de diméthicodiéthylbenzal ; copolymère 3-(4-(2,2-bis-éthoxycarbonylvinyl)-phénoxy)propényl)-méthoxysiloxane/diméthylsiloxane ; dioctylbutylamidotriazone (INCI : Diethylhexyl-Butamidotriazone) ; 2,4-bis-[5-1(diméthyl-propyl)benzoxazol-2-yl-(4-phényl)-imino]-6-(2-éthylhexyl)-imino-1,3,5-triazine (CAS n° 288254-16-0) ; ester tris-(2-éthylhexylique) de l'acide 4,4',4"-(1,3,5-triazine-2,4,6-triyltriimino)-tris-benzoïque) (aussi : 2,4,6-tris-[anilino-(p-carbo-2'-éthyl-1'-hexyloxy)]-1,3,5-triazine (INCI : Ethylhexyl Triazone) ; 2,4-bis-{[4-(2-éthyl-hexyloxy)-2-hydroxy]-phényl}-6-(4-méthoxyphényl)-1,3,5-triazine (INCI : Bis-Ethylhexyloxyphenol Methoxyphenyl Triazine) ; 2,4,6-tris-(biphényle)-1,3,5-triazine ; 2,4-bis-(4'-dinéopentylaminobenzalmalonate)-6-(4"-butylaminobenzoate)-s-triazine ; mérocyanines choisies dans le groupe des composés

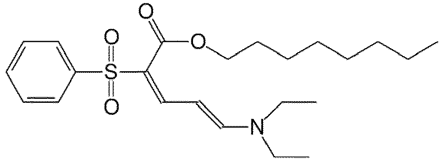

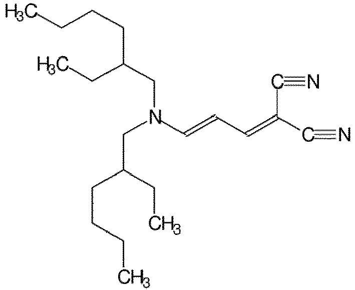

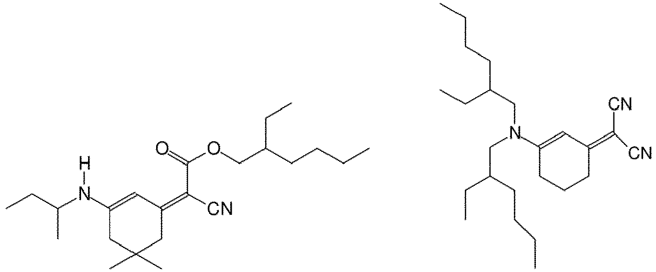

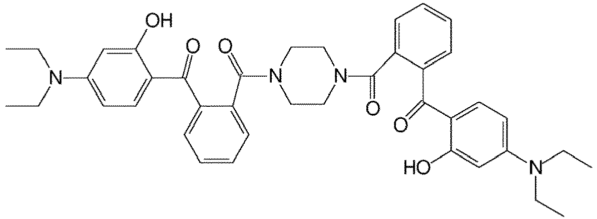

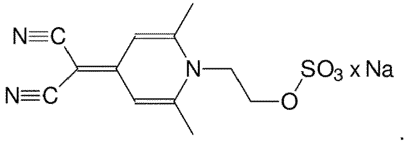

4. Émulsion cosmétique selon l'une des revendications précédentes, **caractérisée en ce que** la préparation est exempte de polymères et de copolymères de l'acide acrylique et de la vinylpyrrolidone.

5. Émulsion cosmétique selon l'une des revendications précédentes, **caractérisée en ce que** la préparation contient du diisostéarate de polyglycéryle-4.

6. Préparation cosmétique selon l'une des revendications précédentes, **caractérisée en ce que** la préparation est exempte d'acide lactique et de ses sels (lactates).

7. Émulsion cosmétique selon l'une des revendications précédentes, **caractérisée en ce que** la préparation contient de l'éthanol.

8. Émulsion cosmétique selon l'une des revendications précédentes, **caractérisée en ce que** la préparation contient une ou plusieurs huiles choisies dans le groupe des composés palmitate d'isopropyle, benzoate d'alkyle en C12-15, dicaprylyléther.

9. Émulsion cosmétique selon l'une des revendications précédentes, **caractérisée en ce que** la préparation contient de l'huile de ricin hydrogénée (INCI : Hydrogenated Rapeseed Oil).

10. Émulsion cosmétique selon l'une des revendications précédentes, **caractérisée en ce que** la préparation contient du dioxyde de titane, qui est revêtu de silice (dioxyde de silicium et/ou acide silicique), qui sur la face extérieure de la couche de silice (c'est-à-dire sur la face opposée au dioxyde de titane) comprend une couche de diméthicone, de siméthicone ou de méthicone.

11. Émulsion cosmétique selon l'une des revendications précédentes, **caractérisée en ce que** le dioxyde de titane utilisé se présente sous la structure cristalline rutile.

12. Émulsion cosmétique selon l'une des revendications précédentes, **caractérisée en ce que** l'oxyde de zinc est revêtu de triéthoxycaprylylsilanes.

13. Émulsion cosmétique selon l'une des revendications précédentes, **caractérisée en ce que** la préparation contient un mélange de dioxyde de titane et d'oxyde de zinc.

14. Émulsion cosmétique selon l'une des revendications précédentes, **caractérisée en ce que** le dioxyde de titane présente une granulométrie primaire de 2 à 100 nm.

15. Émulsion cosmétique selon l'une des revendications précédentes, **caractérisée en ce que** l'oxyde de zinc présente une granulométrie primaire inférieure à 150 nm.
